## Europäisches Patentamt

## European Patent Office

## Office européen des brevets

(19)

(11) Veröffentlichungsnummer: **0 220 656**
**B1**

(12)

# EUROPÄISCHE PATENTSCHRIFT

(45) Veröffentlichungstag der Patentschrift:
19.04.89

(51) Int. Cl.⁴: **C07C 143/44**, C07C 139/00

(21) Anmeldenummer: 86114544.9

(22) Anmeldetag: **21.10.86**

(54) Kontinuierliches Verfahren zur Herstellung von Acyloxybenzolsulfonsäuren.

(30) Priorität: **26.10.85 DE 3538141**

(43) Veröffentlichungstag der Anmeldung:
06.05.87 Patentblatt 87/19

(45) Bekanntmachung des Hinweises auf die Patenterteilung:
19.04.89 Patentblatt 89/16

(84) Benannte Vertragsstaaten:
**AT BE CH DE FR GB IT LI NL SE**

(56) Entgegenhaltungen:
**EP-A- 0 125 641**
**US-A- 3 394 155**
**US-A- 3 503 888**

(73) Patentinhaber: **HOECHST AKTIENGESELLSCHAFT,
Postfach 80 03 20, D-6230 Frankfurt am Main 80(DE)**

(72) Erfinder: **Wellbrock, Werner, Dr., Drei-Linden-Strasse 30,
D-6232 Bad Soden am Taunus(DE)**
Erfinder: **Studeneer, Adolf, Dr., Hölderlinstrasse 52,
D-6233 Kelkheim Taunus)(DE)**

ACTORUM AG

**Beschreibung**

Acyloxybenzolsulfonsäuren bzw. ihre Salze sind seit langem bekannte Verbindungen mit tensidischen Eigenschaften, die zudem gemäß EP-A 98 021 als Perborataktivatoren Verwendung finden können. Zur Herstellung dieser Verbindungen sind eine Reihe von Verfahren bekannt (FR-A 2 559 768, FR-A 2 559 769, EP-A 105 672, EP-A 105 673, EP-A 125 641, US-A 3 394 155, US-A 3 503 888). Diese Verfahren verlaufen jedoch nicht kontinuierlich. Es stellte sich daher die Aufgabe, ein für die großtechnische Herstellung solcher Acyloxybenzolsulfonsäuren, insbesondere für die Herstellung von Isononanoylbenzolsulfonsäure, geeignetes, kontinuierlich arbeitendes Verfahren zu entwickeln.

Gegenstand der Erfindung ist ein Verfahren zur kontinuierlichen Herstellung von $C_7$–$C_{12}$-Acyloxybenzolsulfonsäuren, das darin besteht, daß man Phenol und ein Sulfonierungsreagenz verdünnt mit einer $C_7$–$C_{12}$-aliphatischen Carbonsäure, am Kopf eines auf 80 bis 100°C aufgeheizten Durchlaufreaktors aufgibt und die am unteren Ende dieses Durchlaufreaktors austretende Mischung von Phenolsulfonsäure und der aliphatischen Carbonsäure sowie ein Chlorierungsreagenz am Kopf eines zweiten Durchlaufreaktors aufgibt, wobei durch beide Durchlaufreaktoren Stickstoff im Gegenstrom geleitet wird.

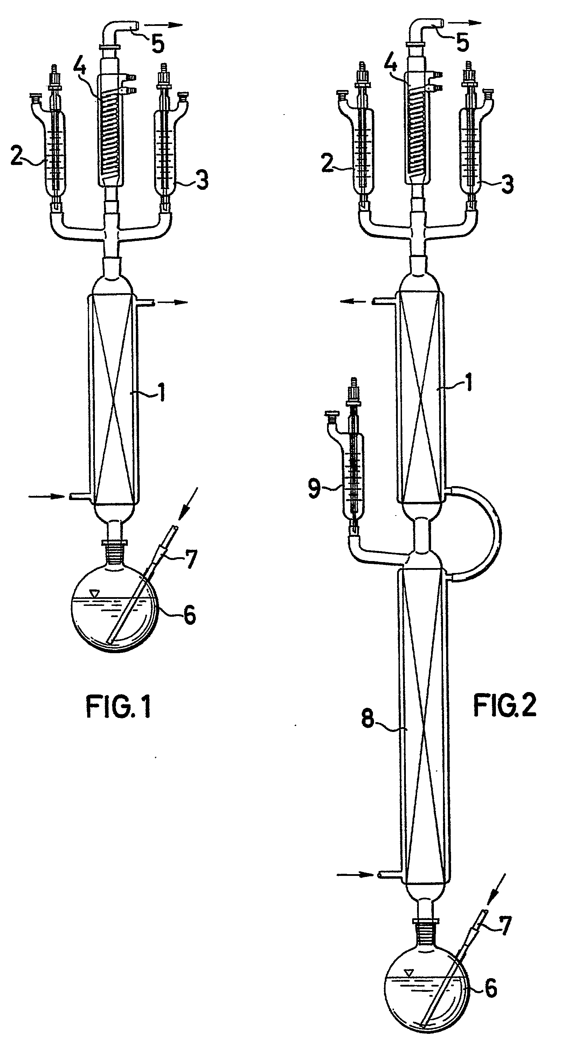

Bevorzugt wird nach diesem Verfahren Isononanoyloxybenzolsulfonsäure hergestellt. Eine zur Herstellung dieses Produkts geeignete Apparatur ist in den Figuren 1 und 2 dargestellt. In einer ersten Stufe (Figur 1) wird zunächst Phenol mit einem Sulfierungsreagenz beispielsweise mit Schwefelsäure oder Oleum, vorzugsweise mit Chlorsulfonsäure, in einem Durchlaufreaktor 1 sulfiert. Beide Ausgangsverbindungen werden am oberen Ende aus zwei Dosiertrichtern 2 und 3 auf den Durchlaufreaktor gegeben, der außerdem noch mit einem Rückflußkühler 4 und einem Gasableitungsrohr 5 versehen ist. Zweckmäßigerweise werden beide Ausgangsverbindungen mit Isononansäure verdünnt und zwar in solchem Verhältnis, daß in beiden Dosiertrichtern ungefähr gleiche Volumina vorliegen. Die Gesamtmenge an Isononansäure entspricht ungefähr der theoretischen Menge dieser Säure, die in der zweiten Stufe zur Veresterung der Phenolsulfonsäure notwendig ist. Der Durchlaufreaktor in dieser ersten Stufe wird auf eine Temperatur von 80 bis 100°C, vorzugsweise 95°C geheizt. Die so hergestellte Phenolsulfonsäure, gelöst in Isononansäure, wird am unteren Ende des Durchlaufreaktors in einer Vorlage 6 gesammelt. Durch den Durchlaufreaktors wird ständig ein Inertgasstrom geführt. Der Einlaß 7 für dieses Inertgas ist so gelegt, daß das Inertgas auch die Phenolsulfonsäure in der Vorlage durchperlt.

Zur Veresterung wird diese Phenolsulfonsäure gelöst in Isononansäure, über einen Dosiertrichter am Kopf eines zweiten Durchlaufreaktors aufgegeben. Man kann aber auch auf die Zwischenisolierung der Phenolsulfonsäure wie es in Figur 1 dargestellt ist, verzichten und arbeitet gemäß der Vorrichtung in Figur 2. Hierbei ist der erste Durchlaufreaktor direkt mit dem zweiten Durchlaufreaktor 8 verbunden und das Gemisch aus Phenolsulfonsäure und Isononansäure tritt unmittelbar in den zweiten Durchlaufreaktor über. Für die hier stattfindende Veresterung wird noch ein Chlorierungsreagenz, vorzugsweise Thionylchlorid benötigt, das aus einem weiteren Dosiertrichter 9 in den zweiten Durchlaufreaktor gegeben wird. Die Menge an Thionylchlorid pro Mol Phenylsulfonsäure beträgt 1 Mol, doch ist auch ein Überschuß bis zu 10% möglich. Die Temperatur in diesem Durchlaufreaktor soll 45 bis 65°C, vorzugsweise 55°C betragen.

Beispiel

In der Vorrichtung gemäß Figur 1 wurden in den beiden Dosiertrichtern jeweils eine Mischung aus 178,8 g (1,53 Mol) Chlorsulfonsäure und 136,5 g (0.86 Mol) Isononansäure bzw. 143 g (1,52 Mol) Phenol und 104 g (0,66 Mol) Isononansäure vorgelegt und in der Weise in den Durchlaufreaktor eindosiert, daß die Verweilzeit ca. 7 Minuten beträgt. Die gesamte Zeit, um die Ausgangsverbindungen in den Reaktor einzudosieren, betrug ca. 2 Stunden. Der Reaktor, der mit einer thermostatisch geregelten Heizung auf 95°C erhitzt wurde, hatte eine Länge von 60 cm und war mit Glasringen gefüllt. Während der gesamten Reaktionszeit wurden 10 Liter Stickstoff pro Stunde durch die Vorlage und durch den Reaktor geleitet. Man erhielt in einer Ausbeute von 93,3% eine mit Isononansäure verdünnte rohe Phenolsulfonsäure folgender Zusammensetzung:
39,7% 4-Phenolsulfonsäure,
10,9% 2-Phenolsulfonsäure,
weniger als 0.05 Hydroxybenzol-2,4-disulfonsäure,
1,8% Phenol

Zur Veresterung wurde dieses Gemisch der rohen Phenolsulfonsäure mit der Isononansäure in einen Dosiertrichter einer Vorrichtung derselben Art wie in Figur 1 dargestellt, gegeben. In den zweiten Dosiertrichter wurden 110,1 g Thionylchlorid gegeben. Beide Komponenten wurden nun so in den Reaktor eingegeben, daß die Verweildauer ca. 50 Minuten betrug. Die Zeit, die dabei benötigt wurde, um die Reaktionskomponenten in den Reaktor einzudosieren, betrug ca. 170 Minuten. Der Reaktor hatte eine Länge von 220 cm, war mit Glasringen vom Durchmesser 6 mm gefüllt und wurde auf eine Temperatur von 55°C erwärmt. Durch die Vorlage zum Auffangen der entstandenen Isononanoyloxybenzolsulfonsäure und dementsprechend auch durch Durchlaufreaktor wurden 10 Liter Stickstoff pro Stunde geleitet. Man erhielt 270 g Isononanoyloxybenzolsulfonsäure der folgenden Zusammensetzung:
63,9% 4-Isononanoyloxybenzolsulfonsäure,
11,5% 2-Isononanoyloxybenzolsulfonsäure,
2,4% 4-Phenolsulfonsäure,
1,2% 2-Phenolsulfonsäure,
1,6% Hydroxybenzol-2,4-disulfonsäure

**Patentansprüche**

1. Verfahren zur kontinuierlichen Herstellung von $C_7$–$C_{12}$-Acyloxybenzolsulfonsäuren, dadurch

gekennzeichent, daß man Phenol und ein Sulfonierungsreagenz verdünnt mit einer $C_7$–$C_{12}$-aliphatischen Carbonsäure, am Kopf eines auf 80 bis 100°C aufgeheizten Durchlaufreaktors aufgibt und die am unteren Ende dieses Durchlaufreaktors austretende Mischung von Phenolsulfonsäure und der aliphatischen Carbonsäure sowie ein Chlorierungsreagenz am Kopf eines zweiten Durchlaufreaktors aufgibt, wobei durch beide Durchlaufreaktoren Stickstoff im Gegenstrom geleitet wird.

2. Verfahren nach Anspruch 1, dadurch gekennzeichnet, daß man Isononanoyloxybenzolsulfonsäure herstellt.

## Claims

1. A process for the continuous production of $C_7$–$C_{12}$-acyloxybenzenesulfonic acids, which comprises charging phenol and a sulfonating agent, diluted with an aliphatic $C_7$–$C_{12}$-carboxylic acid, to the top of a continuous-flow reactor heated to 80–100°C and charging the mixture of phenolsulfonic acid and the aliphatic carboxylic acid leaving the bottom end of this continuous-flow reactor, and a chlorinating agent to the top of a second continuous-flow reactor, nitrogen being passed in counter-current through both continuous-flow reactors

2. The process as claimed in claim 1, wherein isononanoyloxybenzenesulfonic acid is produced.

## Revendications

1. Procédé pour préparer en continu des acides acyloxy-benzène-sulfoniques à acyles en $C_7$–$C_{12}$, procédé caractérisé en ce qu'on introduit le phénol et un agent de sulfonation, dilués avec un acide carboxylique aliphatique en $C_7$–$C_{12}$ à la tête d'un réacteur de passage chauffé à une température de 80 à 100°C, et on introduit à la tête d'un second réacteur de passage le mélange qui sort à la partie inférieure du premier réacteur de passage et qui est constitué d'acide hydroxy-benzène-sulfonique et de l'acide carboxylique aliphatique ainsi qu'un agent de chloration, en même temps qu'on fait passer de l'azote à contre-courant à travers les deux réacteurs.

2. Procédé selon la revendication 1 caractérisé en ce qu'on prépare l'acide isononanoyloxy-benzènesulfonique.

FIG. 1

FIG. 2